(19) Europäisches Patentamt
European Patent Office
Office européen des brevets

(11) **EP 3 253 291 B1**

(12) **EUROPEAN PATENT SPECIFICATION**

(45) Date of publication and mention
of the grant of the patent:
**16.06.2021 Bulletin 2021/24**

(21) Application number: **16708755.0**

(22) Date of filing: **22.01.2016**

(51) Int Cl.:
**A61B 6/00** *(2006.01)*

(86) International application number:
**PCT/US2016/014413**

(87) International publication number:
**WO 2016/126437 (11.08.2016 Gazette 2016/32)**

(54) **AUTO-POSITIONING FOR IN-ROOM RADIOGRAPHY APPARATUS**

AUTOMATISCHE POSITIONIERUNG FÜR RAUMINTERNE RADIOGRAFIEVORRICHTUNG

POSITIONNEMENT AUTOMATIQUE POUR APPAREIL DE RADIOGRAPHIE EN SALLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.02.2015 US 201514611298**

(43) Date of publication of application:
**13.12.2017 Bulletin 2017/50**

(73) Proprietor: **Carestream Health, Inc.
Rochester, NY 14608 (US)**

(72) Inventors:
• **LALENA, Michael, C.
Rochester, NY 14608 (US)**
• **PETRUCCI, John, L.
Rochester, NY 14608 (US)**

(74) Representative: **Klang, Alexander H.
Wagner & Geyer Partnerschaft mbB
Patent- und Rechtsanwälte
Gewürzmühlstrasse 5
80538 München (DE)**

(56) References cited:
WO-A1-2014/033614       JP-A- 2006 014 754
JP-A- 2008 161 593       JP-A- 2013 154 146
JP-A- 2014 117 368       US-A1- 2005 041 768
US-A1- 2011 122 995

**Description**

**TECHNICAL FIELD**

The disclosure relates generally to the field of radiography and more particularly to methods and apparatus for automatically positioning the x-ray source and detector based on exam type and patient characteristics.

**BACKGROUND**

In-room radiography apparatus are used for acquiring numerous types of radiographic images, taken from various portions of patient anatomy, for patients of various sizes, ages, and conditions. In a typical room radiography environment, technicians routinely switch between any of more than 50 types of exams, reconfiguring the imaging apparatus for each patient. Some amount of equipment setup time is required for each exam, as the patients are positioned and as the placement of the x-ray source and receiver are adjusted appropriately for each patient and for each exam type.

In conventional practice, some amount of automated positioning or pre-positioning may be provided, so that the operator or technician is provided with a reasonable starting point for carrying out each type of exam that is needed. Setup for each type of exam is performed by a time-consuming process of manually positioning the x-ray source and detector in place for the exam and recording these positions. Then, when a specific exam is requested, equipment can be automatically moved into the programmed position according to operator or technician instructions. A number of variables need to be stored for conventional setup, with the values relative to an origin position in the x-ray room.

Using conventional setup and recording methods for equipment positioning, equipment setup and programming is site-specific. Even where two radiography sites may have the same room size and other common characteristics, there are typically slight differences in how equipment is positioned in each site. The reference origin (0,0,0 in Cartesian coordinates) is generally one corner of the room that houses the radiography equipment. Movement for any of the various apparatus in the room is referenced to the reference origin for subsequent positioning. The coordinates for each exam must be individually programmed at each site, since the referenced position lies outside the radiography apparatus itself.

Although conventional practices provide a solution that enables some level of automation for initial apparatus positioning, these existing techniques have proven to be relatively cumbersome and costly. The sequence needed for setup and recording positions for each exam is time-consuming and it can take several hours for a service engineer and operations staff to work through a listing of prospective exams and configure the equipment for each exam type and for imaging patients of various heights and builds. As noted earlier, there is no benefit gained from setup of earlier installations; each site must be individually set up and its results programmed. Moreover, existing setup practices fail to adapt to different height and overall physical make-up of the patient population. The programmed setup parameters can be a poor match for patients who are shorter or taller than average. Additional setup work is often required to further adjust apparatus positioning to suit patient size and position after the x-ray source and detector are moved to their original positions.

Attention is drawn to US 2011 122 995 A1, which relates to systems and methods for remote diagnostic imaging. The systems include a diagnostic imaging kiosk including a first housing module and a second housing module. The first housing module is configured to house, among other things, a diagnostic imaging system (e.g., an X-ray system). The second housing module is configured to house electronics associated with the operation, control, and networking of the kiosk. The electronics include, for example, a primary controller, an X-ray controller, an X-ray generator, an internal display controller, an external display controller, one or more routers, and a digital radiology module. The kiosk is configured to communicatively connect to a remote technician's workstation, and a remote technician at the workstation is able to remotely control the kiosk through a packet-switched network. The control of the kiosk inter alia includes controlling access to the kiosk, the height position of a diagnostic imaging unit, the capture of diagnostic images and the display of information to a patient. Further, JP 2013 154 146 A relates to a radiographic imaging system capable for setting a number of images to be taken during a long-length imaging process. The radiographic imaging system includes calculation means for calculating the number of images to be taken in the long-length imaging on the basis of a region ROI of a subject and the size of a detection part P in the radiographic imaging device, and a position adjusting means for adjusting the position of the radiographic imaging device for each imaging in the long-length imaging.

Thus, it can be appreciated that there is a need for an auto-positioning utility to assist setup and configuration of a radiography site

Document WO2014033614 A1 may be considered to disclose an auto-positioning apparatus for an x-ray imaging system, the auto-positioning apparatus comprising: a first transport apparatus configured to automatically move and position an x-ray source according to three spatial xyz coordinates relative to a detector in response to receiving the three spatial xyz coordinates; and a processor communicatively coupled to the first transport apparatus, the processor programmed to: retrieve electronically stored data identifying an exam type; retrieve electronically stored patient data including data identifying a height of a patient to be imaged; determine a current position of the x-ray source along each of the three spatial xyz coordinates and determine a source imaging position along each of the three spatial xyz coordinates according to the retrieved data identifying the exam type and the patient data; and provide a first positioning signal identifying the three spatial xyz coordinates to the first transport apparatus to automatically move the x-ray source from the determined current position to the determined source imaging position.

## SUMMARY

It is an object of the present disclosure to advance the art of radiography. A related object of the present disclosure is to address the need for an auto-positioning utility that does not require setup procedures for every possible type of exam and that further adapts to patient height and size characteristics. An advantage offered by methods of the present disclosure is straightforward customization of a radiographic system for patient height based on the anatomy of a local or regional population.

These objects are given only by way of illustrative example, and such objects may be exemplary of one or more embodiments of the invention. Other desirable objectives and advantages inherently achieved by the may occur or become apparent to those skilled in the art. The invention is defined by the appended claims. In accordance with the present invention, an apparatus and a method as set forth in claims 1 and 9 is provided. Further embodiments are inter alia disclosed in the dependent claims.

According to one aspect of the disclosure, there is provided an auto-positioning apparatus for an x-ray imaging system, inter alia comprising:

> a transport apparatus that is configured to position an x-ray source relative to a detector; and
> a computer processor that is configured to:

>> (i) accept exam data that indicates an exam type for the patient;
>> (ii) acquire patient data that is indicative of patient height;
>> (iii) determine the present position of the transport apparatus and determine an intended imaging position according to the accepted exam data and acquired patient data; and
>> (iv) provide a positioning signal to move the transport apparatus from its present position to the intended imaging position.

## BRIEF DESCRIPTION OF THE DRAWINGS

The foregoing and other objects, features, and advantages of the invention will be apparent from the following more particular description of the embodiments of the invention, as illustrated in the accompanying drawings.

The elements of the drawings are not necessarily to scale relative to each other.

> FIG. 1 is a diagram that shows a perspective view of a radiography apparatus that uses a processor and stored information or data in order to position the x-ray source and detector appropriately for each exam and patient type.
> FIG. 2 is a perspective view that shows radiation directed toward a wall stand bucky in a vertical orientation.
> FIG. 3 is a perspective view that shows radiation directed toward a wall stand bucky in a horizontal orientation.
> FIG. 4 is a perspective view that shows radiation directed toward a table bucky.
> FIG. 5 is a perspective view that shows the radiation source and image detector in an initial reference position according to an embodiment of the present disclosure.
> FIG. 6 is a side view that shows positioning of imaging components for a particular imaging example.
> FIG. 7 is a schematic diagram that shows imaging components used for exam setup.
> FIG. 8 is a logic flow diagram that shows a sequence for acquiring images according to an embodiment of the present disclosure.

## DETAILED DESCRIPTION OF THE EMBODIMENTS

The following is a detailed description of the preferred embodiments, reference being made to the drawings in which the same reference numerals identify the same elements of structure in each of the several figures.

Various relative terms such as "above," "below," "top," "bottom," "height," "depth," "width," and "length," etc. may be used in the present disclosure to facilitate description of various embodiments. The relative terms are defined with respect to a conventional orientation of a structure and do not necessarily represent an actual orientation of the structure in manufacture or use. The following detailed description is, therefore, not to be taken in a limiting sense.

Where they are used, the terms "first", "second", and so on, do not necessarily denote any ordinal or priority relation, but may be used for more clearly distinguishing one element or time interval from another.

As used herein, the term "energizable" relates to a device or set of components that perform an indicated function upon receiving power and, optionally, upon receiving an enabling signal. The opposite state of "energizable" is "disabled".

The term "actuable" has its conventional meaning, relating to a device or component that is capable of effecting an action in response to a stimulus, such as in response to an electrical signal, for example.

The term "modality" is a term of art that refers to types of imaging. Modalities for an imaging system may be conventional x-ray, fluoroscopy, tomosynthesis, tomography, ultrasound, MMR, or other types of imaging. The term "subject" refers to the patient who is being imaged and, in optical terms, can be considered equivalent to the "object" of the corresponding imaging system.

In the context of the present disclosure, the term "coupled" is intended to indicate a mechanical or electrical association, connection, relation, or linking, between two or more components, such that the state of one component affects the spatial disposition of a component to which it is coupled. For mechanical coupling, two components need not be in direct contact, but can be linked through one or more intermediary components or fields.

It will be understood that when an element is referred to as being "connected," or "coupled," to another element, it can be directly connected, communicatively connected, or coupled to the other element or intervening elements or magnetic fields may be present. In contrast, when an element is referred to as being "directly connected," or "directly coupled," to another element, there are no intervening elements present. Other words used to describe the relationship between elements should be interpreted in a like fashion (e.g., "between," versus "directly between," "adjacent," versus "directly adjacent," etc.).

The term "exemplary" indicates that the description is used as an example, rather than implying that it is an ideal.

The term "in signal communication" as used in the application means that two or more devices and/or components are capable of communicating with each other in at least one direction via signals that travel over a signal path. Signal communication may be wired or wireless. The signals may be communication, power, data, or energy signals which may communicate information, data, power, and/or energy from a first device and/or component to a second device and/or component along a signal path between the first device and/or component and second device and/or component. The signal paths may include physical, electrical, magnetic, electromagnetic, optical, wired, and/or wireless connections between the first device and/or component and second device and/or component. The signal paths may also include additional devices and/or components between the first device and/or component and second device and/or component.

The statistical term "patient population" relates to a set of a plurality of patients from which statistical information can be obtained. Patient population-related data, such as average patient height, can be calculated from the set or a subset of patients that have been examined at a site or can be obtained from information about the patients who are most likely to be served at a particular imaging site.

The term "exam type" is associated with a body part that is to be imaged.

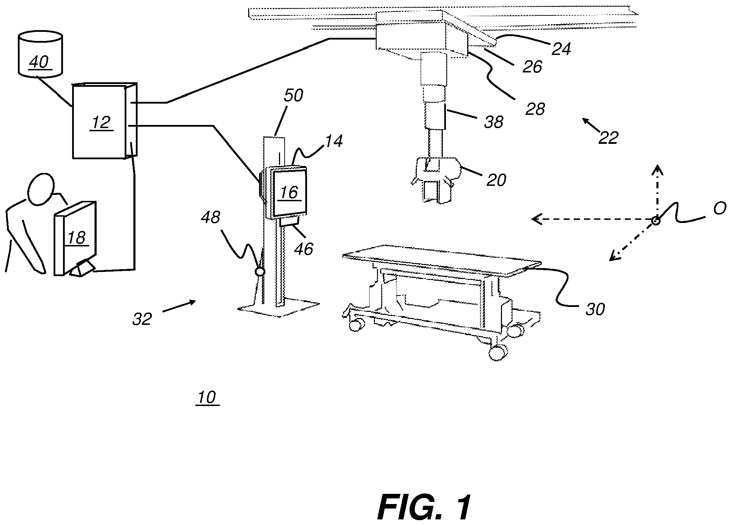

The perspective view of FIG. 1 shows a radiography apparatus 10 that uses a processor 12 and electronically stored information in order to position the x-ray source and detector appropriately for each exam and patient type. A detector 16, such as a digital radiography (DR) detector or a film cassette or computed radiography (CR) cassette provides a receptive medium for incident radiation obtained from an x-ray source 20. Detector 16 mounts on a wall stand 50 that has a bucky 14 or may be placed behind the patient when lying on a bed 30. Wall stand 50 can be mounted on the floor or, alternately, suspended from the ceiling or other suitable structure. Bucky 14 holds detector 16 and typically also holds a grid. X-ray source 20 mounts on a transport apparatus 22 that includes a ceiling mount apparatus 28 in FIG. 1, for movement to different positions within the room. Ceiling mount apparatus 28 may move along a single line or moves along a plane that is substantially in parallel with the ceiling.

Transport apparatus 22 in FIG. 1 has an optional telescoping arm section 38 for raising or lowering x-ray source 20 appropriately. Transport apparatus 22 is in signal communication with processor 12 and has an actuator 26 that controls apparatus 22 movement and a sensor 24 that indicates apparatus 22 position. Bucky 14 also has a sensor 48 and an actuator 46 that report and control the position of detector 16 as part of a detector transport apparatus 32. Processor 12 has a display 18 that serves as an operator interface for instruction entry, data entry, control data input, and monitoring. Processor 12 is coupled to and is in signal communication with actuators 26 and 46 and with various sensors 24 and 48 for both the x-ray source 20 and detector 16 or bucky 14, and tracks and stores position settings used to generate each type of image. Processor 12 also is in signal communication with a patient database 40, such as a DICOM database, for retrieving and storing patient data and image data.

In conventional practice, the radiography apparatus 10 has an origin O that is established in an initial setup procedure and that is typically considered to be at one corner of the radiography room. Any setup position for the components of apparatus 10 may then be expressed and stored with reference to the origin O. By identifying the spatial coordinates of a component in a particular position in relation to origin O, computer processor 12 can identify the distance needed to move that component to another desired position which has its own identifying spatial coordinates. For each of numerous exam types, component positioning for each element of the system must be individually identified and electronically stored, requiring hours of laborious positioning, testing, and storage procedures.

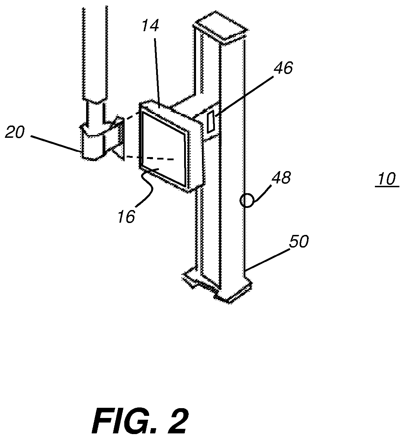

FIGs. 2, 3, and 4 show different arrangements of x-ray source 20 for imaging. FIG. 2 shows a conventional bucky mounting arrangement in a floor-mounted wall stand 50, in which bucky 14 serves as the mounting and transport mechanism for detector 16. In the FIG. 2 configuration, actuator 46 of bucky 14 moves detector 16 up and down. Sensor 48 reports the position of bucky 14, and thus of detector 16, relative to wall stand 50 height.

It should be noted that sensor 48 can have various arrangements for providing information about bucky 14 motion, with different configurations available according to actuator 46 type, such as when actuator 46 is a stepper motor, since the stepper motor can inherently store an origin position and uses step actuations for controlled incremental movement relative to the origin position. Multiple sensors 48 can be used to detect absolute position or relative position and position changes. According to an embodiment of the present disclosure, a radiography apparatus 10 uses a pair of encoders for sensing bucky 14 position. An optical encoder monitors drive shaft motion of the actuator motor and provides relative motion information when the motor is actuated. Another sensor provides absolute position information, such as a potentiometer, Hall sensor, or magnetic sensor, for example. Absolute position information establishes a baseline reference after power loss or at other times when it is necessary or useful to establish or re-establish a "home" reference position. It can be appreciated that any of a number of encoder arrangements are possible and can be implemented by those skilled in the motion control sensing arts. Other encoders and sensors, for example, can be used to detect rotation angle.

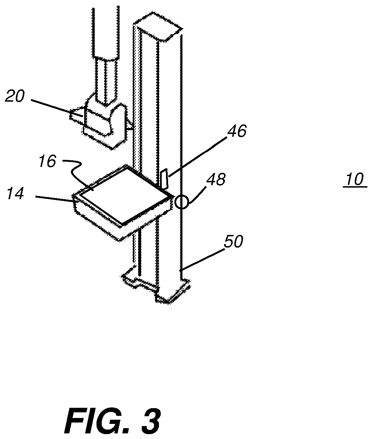

FIG. 3 shows an alternate configuration using bucky 14 for imaging of a hand, shoulder, extended arm, or other extremity placed on detector 16 that is disposed in a horizontal orientation. In this type of configuration, the height of bucky 14 and thus detector 16 can be adjusted. The rotation angle of bucky 14 can also be tracked using one or more rotational encoders.

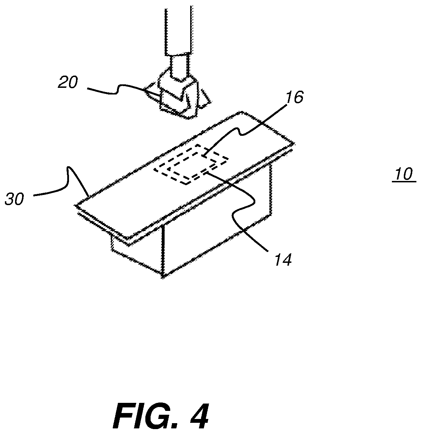

FIG. 4 shows yet another alternate configuration wherein x-ray source 20 is directed toward bed 30. X-ray detector 16 is placed beneath the patient, as indicated in dashed outline in FIG. 4. With a bed arrangement, detector 16 is variably positioned horizontally with respect to the patient; a table bucky 14 may be used, as also indicated in dashed outline. Bed 30 is moved in order to position the anatomy of interest beneath x-ray source 20 for imaging.

As is clear from FIGs. 1-4, each imaging arrangement requires movement of the x-ray source 20 to some position relative to the detector 16, which may also be moved to a different position, such as to a vertical or horizontal position in a bucky or beneath a bed, for example. Both transport apparatus 22 and 32 can be used. For each position, reference to some type of origin may be used. In conventional practice, reference is to some fixed-origin O point in the radiography room, such as a corner of the room as described previously. Some embodiments of the present invention may or may not use a spatial position of the room itself as the reference, but establish a different origin as the reference point, wherein the origin relates to the position of detector 16 and/or source 20 relative to the patient's height and is used to measure motion of the x-ray source 20 relative to the radiography detector 16.

Unlike conventional positioning methods, embodiments of the present disclosure use the position of the detector 16 as an origin for corresponding movement of x-ray source 20. This alternative method simplifies the process of installation setup for the x-ray system at a particular site and allows the use of electronically stored tabular information that relates to patient anatomy for providing auto-positioning.

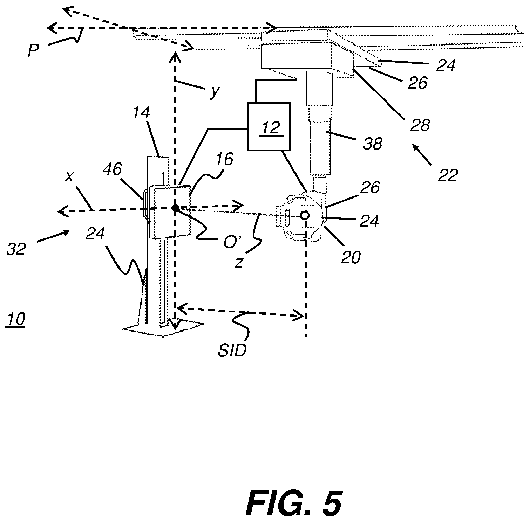

FIG. 5 is a perspective view that shows the radiation source and image detector in an initial reference position according to an embodiment of the present disclosure. Processor 12 provides the same basic actuation control and tracking functions described with reference to the FIG. 1 embodiment, as well as connections to patient medical database 40. With this arrangement, an origin O' is established as a point on detector 16. Detector 16 is planar, in the x-y plane as represented in FIG. 5. An orthogonal vector z is shown extending from the x-y plane at origin O'. Using a straightforward transform matrix, X-ray source 20 position and angle can be computed with reference to origin O' along the three dimensional x, y, and z reference axes. Transport apparatus 22 can be referenced to the same origin O', using one or more reference axes, as it is moved along the plane P of the ceiling. A source-to-image distance (SID) is defined between the radiation point source of x-ray source 20 and origin O' on detector 16.

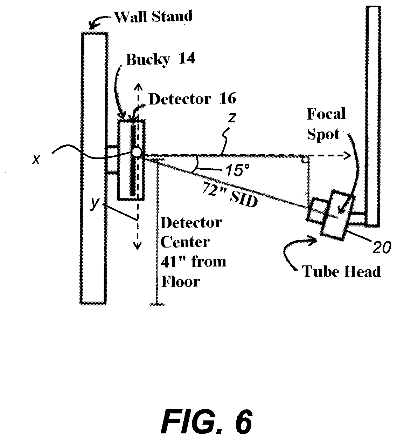

FIG. 6 shows, from a side view, how x-ray source 20 can be located with respect to detector 16 when source 20 is directed toward detector 16 at an angle. Here, a 72-inch SID is needed, with x-rays directed along a 15-degree angle toward detector 16.

Origin O' can be referenced to the detector 16 because processor 12 of radiography apparatus 10 is able to track movement of detector 16 and source 20 relative to the actuators 26, 46 that are energized to move them and sensors 24, 48 that provide feedback to the processor that indicates spatial position or angle based on spatial coordinates using one or more of the reference axes.

Calibration procedure

In order to calibrate radiography apparatus 10 so that its various sensors 24, 48 and actuators 26, 46 have a common reference, a single positioning procedure may be all of the positional calibration setup that is needed as part of installation. The arrangement of FIG. 5 shows how this reference can be established for a standard x-ray system setup. Among other variables, this setup identifies boundaries of the room and positions of components at the site that may present obstacles to movement of the x-ray source such as wall stand and table, for example. The operator adjusts detector 16 to a suitable height, then aims x-ray source 20 directly toward detector 16 from a predetermined SID. An operator instruction sequence, entered once during initial installation of apparatus 10, then causes processor 12 to electronically store the established reference position in a processor accessible storage location. For the exemplary embodiment of FIG. 5, with x-ray source 20 aimed at an orthogonal to detector 16, this means storing the following exemplary data:

(i) position of transport apparatus 22 and ceiling mount apparatus 28 along the ceiling;
(ii) extension of x-ray source 20 downward from transport apparatus 22;
(iii) angle of source 20; and
(iv) height of detector 16 along bucky 14.

For a C-arm installation, the needed information is somewhat simpler and calibration for room dimensions may not be needed. Position information is readily obtainable from the imaging apparatus itself, such as height of the axis of rotation for source, which relates to patient height, and detector and distance separating source and detector (SID).

Once the reference position is properly entered and stored, movement relative to the reference position can then be automated so that a suitable repositioning of detector 16 and its associated bucky 14 and source 20 can be effected for each type of exam. Significantly, this repositioning can be adapted to the dimensions of the patient, as described in more detail subsequently. A small amount of additional positioning data may also be stored, including parameters related to the location of possible physical obstructions, position of bed 30, preferred tube head orientation, and other data to support arrangements of the imaging components, such as for imaging extremities, for example.

According to an embodiment of the present disclosure, it may be useful or necessary to store additional calibration settings. Factors that determine this include variables in the arrangement of components that are used at a particular site. For example, the bed 30 (FIG. 1) may be parallel or perpendicular to wall stand 50; its relative position may make it necessary to repeat wall calibration at different locations, such as at the head and at the foot of bed 30. Additional calibration data for bed height(s) may also be useful to obtain. Additional calibration may be helpful for wall stands 50 that move along rails on the floor or other surface. For example, the wall stand 50 may not be perfectly vertical, requiring minor calibration adjustment. Bed 30 may not be perfectly horizontal; a ceiling mounted transport apparatus 22 may not be parallel with the surface of bed 30.

With the reference position established, processor 12 can then use tabular information to adjust the position settings or spatial coordinates for radiography apparatus 10 components. Two types of tabular information are of particular value: exam-related data and patient-related data.

Exam-related data

In conventional workflow for radiography, the majority of images that are acquired are from a standard set of images for different exam types. Each of these standard exam types has a standard setup practice. By way of example, Table 1 lists a number of standard exam types for C-spine views and some of the parameters that are typically used for positioning for each exam type.

The type of information provided in Table 1 is typical of information available for standard practice with other exam types. Embodiments of the present disclosure take advantage of this standardized information so that, knowing the starting position of x-ray system components within the x-ray room and the size of the patient, it can be possible to determine the needed movements from the starting position to an imaging position of each component for automating the equipment setup for imaging.

**Table 1. Exemplary Conventional Setup Parameters for C-Spine Views**

| C-Spine Views | Position, Bucky & SID | | X-Ray Angle |
| --- | --- | --- | --- |
| | Preferred Position/Bucky/SID | Alternate Position/Bucky/SID | |
| C-Spine - AP | Erect/Wall/72 in. | Supine/Table/40 in. | 15° Up |
| Odontoid | Supine/Table/40 in. | Erect/Wall/40 in. | 0° Center |
| C-Spine - Dens - AP (Fuchs) | Erect/Wall/72 in. | Supine/ Table/40 in. | 20° Up |
| C-Spine - Lateral | Erect/Wall/72 in. | | 0° Center |

(continued)

| C-Spine Views | Position, Bucky & SID | | X-Ray Angle |
| --- | --- | --- | --- |
| | Preferred Position/Bucky/SID | Alternate Position/Bucky/SID | |
| C-Spine - Lateral - XTable | Supine/Wall/72 in. | Supine/ Table Top/40 in. | 0° Center |
| C-Spine - Lateral Extension | Erect/Wall/72 in. | | 0° Center |
| C-Spine - Lateral Flexion | Erect/Wall/72 in. | | 0° Center |
| C-Spine - LAO | Erect/Wall/72 in. | Prone/Table/40 in. | 15° Up |
| C-spine - RAO | | | |
| C-Spine - LPO | Erect/Wall/72 in. | Supine/Table/40 in. | 0° Center |
| C-Spine - RPO | | | |

Patient-related data

The distribution of specific bones and organs of the human anatomy is known to be generally proportional to the height of the patient, with some further consideration for age, weight, sex, and other factors. Embodiments of the present disclosure take advantage of this proportionality and use patient data where available or use default statistical data from the patient population to more accurately estimate the needed movement or adjustment from a current position to an imaging position of x-ray apparatus components for automatic positioning.

By way of example, and not of limitation, Table 2 lists a number of features of the human frame and their height on an adult of average size. These exemplary values are based on averages for the US population.

For imaging of upper extremities such as hand, fingers, thumb, wrist, elbow, and forearm, table-top imaging is typically used, employing the arrangement of FIG. 3, for example.

Patient height and weight data vary geographically and culturally, so that a single standard set of default values for worldwide use would be impractical. By way of example, heights for different anatomical regions for a typical 70-inch tall adult are listed in Table 3, with weight norms given by example in Table 4. By way of example, average patient heights for various countries are listed in Table 5. Also by way of example, some exemplary standard SID values are shown in Table 6.

**Table 2. Anatomy Height for Adult of Average Height** (US: **70 inches**)

| Anatomy (Exam type) | Height |
| --- | --- |
| Feet | 1" |
| Toes | 1" |
| Calcaneus | 1" |
| Ankle | 4" |
| Tibia / Fibula | 15" |
| Knee | 20" |
| Long Leg | 20" |
| Femur | 28" |
| Hip | 33" |
| Coccyx | 33" |
| Pelvis | 35" |
| Sacrum | 35" |
| Sacroiliac Joints | 38" |
| L-Spine | 40" |
| Abdomen | 44" |

(continued)

| Anatomy (Exam type) | Height |
|---|---|
| KUB | 44" |
| Small Bowel | 44" |
| Large Intestine (BE) | 44" |
| IVP | 44" |
| T/L-Spine | 48" |
| Gallbladder | 48" |
| UGI (Contrast) | 48" |
| Chest | 50" |
| T -Spine | 52" |
| Scoliosis | 52" |
| Humerus | 53" |
| Ribs | 54" |
| Sternum | 54" |
| Sternoclavicular | 58" |
| Esophagus (Contrast) | 58" |
| Shoulder Girdle | 59" |
| C-Spine | 60" |
| Soft Tissue Neck | 62" |
| Mastoids | 62" |
| Mandible | 62" |
| TMJ | 62" |
| Dental | 62" |
| Sinuses | 64" |
| Skull | 65" |
| Nasal Bones | 65" |
| Facial Bones | 65" |
| Orbits | 65" |
| Sella Turcica | 65" |
| Zygomatic Arches | 65" |

**Table 3. Patient Height Norms (US)**

| Patient Size | Patient Height |
|---|---|
| Very Low Birth Weight | 16" |
| Low Birth Weight | 18" |
| Newborn | 20" |
| Infant | 30" |
| Child | 42" |

(continued)

| Patient Size | Patient Height |
|---|---|
| Preadolescent | 48" |
| Adolescent | 66" |
| Small Adult | 66" |
| Medium Adult | 70" |
| Large Adult | 72" |

**Table 4. Patient Weight Norms**

| Patient Size | Age / Weight |
|---|---|
| Very Low Birth Weight | < 1.5 kg |
| Low Birth Weight | < 2.5 kg |
| Newborn | < 1 month |
| Infant | < 2 years |
| Child | < 11 years |
| Preadolescent | < 13 years |
| Adolescent | < 21 years |
| Small Adult | < 50 kg |
| Medium Adult | < 80 kg |
| Large Adult | > 80 kg |

**Table 5. Average Patient Heights by Country**

| Country | Avg. Patient Height |
|---|---|
| US | 70" |
| Germany | 69" |
| Japan | 67" |
| China | 68" |
| Swede | 71" |

**Table 6. Exemplary Standard SID Values**

| SID | Distance |
|---|---|
| Short (US) | 40" |
| Short (Europe) | 100cm |
| Long (US) | 72" |
| Long (Europe) | 180cm |

It should be noted that electronically stored values such as those listed in Tables 1-6 are exemplary and that a number of other values can be stored, including values added or edited at a specific site. For example, the Table or Table Top SID at a particular site may need to be adjusted based on ceiling height limitations. The Horizontal Wall Stand SID might need to be adjusted based on the relationship of wall stand height with ceiling distance. The distance between the patient bed 30 (FIG. 1) and the ceiling may also be a limitation, requiring adjustment of the SID at a particular site.

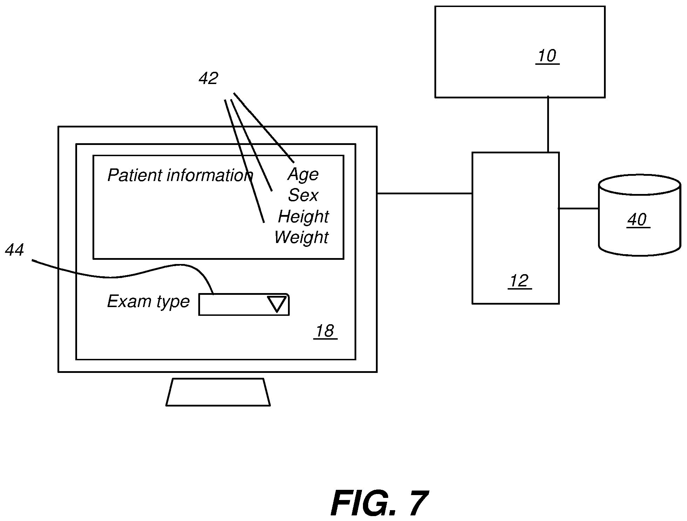

An embodiment of the present disclosure enables automatically initiated positioning of radiography apparatus 10 components using stored information about the exam type and stored information about patient height and build, and related data such as the data listed in Tables 1 through 5, with preferred SID information from Table 6. The schematic diagram of FIG. 7 shows an operator interface presented on display 18 that allows entry or retrieval of patient information relevant to auto-positioning. Patient information fields 42 may be entered directly by an operator or may be obtained from patient records in a database 40, such as a DICOM (Digital Imaging and Communications in Medicine) database, or some other patient database. Default average patient height information can be obtained using a global or regional (by country) default, or using a value entered by an administrator of the system. This patient height would be used if none were provided by the medical record or by the user. Alternately, the operator interface can be used to enter default patient height information directly, such as wherein the default height information has been separately computed.

According to an alternate embodiment of the present disclosure, statistical values such as average patient height data for a particular site are adjusted over time, based on frequency of manual adjustment and trends detected in the adjustment data. For example, where manual adjustment related to patient height is needed in an appreciable number of cases and where the manual adjustment is predominantly downward, the software executed by processor 12 (FIG. 5) adjusts its stored default height values accordingly. This optional statistical adjustment capability can be particularly useful, for example, to reduce the need for manual adjustment where data about the patient population that is served by a particular imaging site may be unavailable or incomplete.

Still referring to FIG. 7, another field 44 accepts an instruction or command entry that specifies the exam type, such as from a pull-down menu, for example. In response to this information, processor 12 sends a sequence of auto-positioning commands to radiography apparatus 10 (FIG 5), controlling movement of transport mechanisms and actuators for positioning the x-ray source 20 and detector 16.

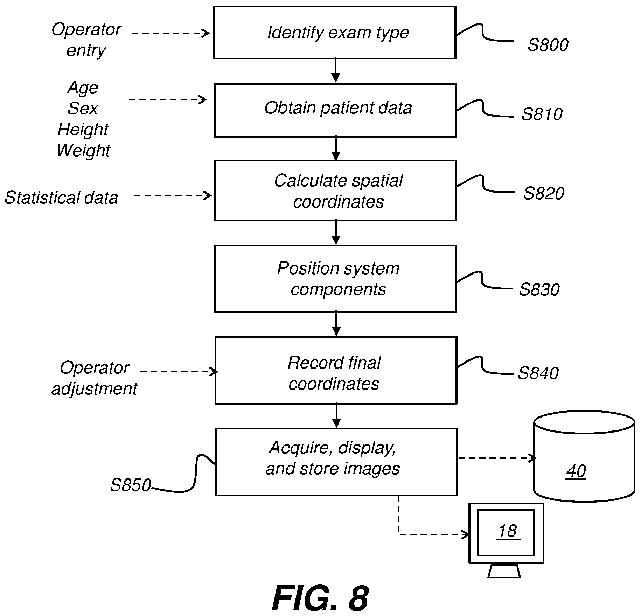

The logic flow diagram of FIG. 8 shows a sequence of steps for image acquisition and display that enable automatic moving and positioning, and alternative final manual positioning adjustment of radiography apparatus 10 components according to an embodiment of the present disclosure. In an exam identification step S800, the type of radiological exam is specified and a corresponding instruction is sent to processor 12. As was shown in FIG. 7, this identification can be provided by a menu selection or other operator entry or instruction via the user interface; alternately, the exam type can be provided from some other system or using a different workflow arrangement. An obtain patient data step S810 then acquires the needed information for auto-positioning, as described, including anatomy information such as that used in conjunction with Tables 1 - 5. Patient information can be obtained from operator entry at an operator interface or from stored electronic patient records data, such as from a Hospital Information Systems (HIS) or Radiological Information Systems (RIS) database, for example.

It should be noted that patient data can include patient height, weight, age, and other information that relates to particular anatomical characteristics of the patient. Where patient data of this type is available, values for patient height can be used directly for auto-positioning computation as described herein. Where patient data is not available or is incomplete, inconsistent, or ambiguous, default statistical values can be used for the auto-positioning computation that follows.

Continuing with the sequence of FIG. 8, once the exam is identified and patient data acquired, a calculation step S820 may be executed. In calculation step S820, previously stored positioning information for the given exam is retrieved from memory circuitry accessible to processor 12. This stored information indicates what equipment setup is used, such as the basic arrangement of FIG. 2, for example, with initial default positions for detector 16 and x-ray source 20. Patient-specific information from step S810 is then used to obtain the desired equipment positioning for the particular patient. The operator may be prompted to request different handling according to available options for the type of imaging selected. This information then goes to processor 12 for calculation step S820 that returns spatial coordinates for each imaging component.

Particularly where patient data is not readily available, step S820 in FIG. 8 can use default statistical data obtained from the patient population, along with data about the patient and exam type from preceding steps, in order to calculate the needed values for auto-positioning. By way of example, and not by way of limitation, the type of exam identified can be a C-Spine LAO exam for a patient who is age 12. Some of the values and positional parameters of particular interest for this imaging type were shown earlier with reference to FIG. 6. The default position for C-Spine LAO is a wall view as in FIG. 2, with a long SID and an X-ray angle of 15 degrees upwards. Detector height must be calculated for auto-positioning using actual patient height data, where available, or using default statistical data.

For this example, with the actual patient height unknown, the detector 16 height for a patient is adjusted from a default adult setting by a height factor, using the following calculation:

$$60 \text{ in. X (height factor)} = 41 \text{ in.}$$

The value of 60 in. for C-Spine imaging is acquired from Table 2. The height factor is computed from statistical Table 3 values as the ratio of Preadolescent Height to Medium Adult height: 48 in. / 70 in.

This calculation thus sets the center of the detector 16 at 41 in. The 15-degree angle and long SID set the tube head height from the floor for X-ray source 20 accordingly:

$$41 \text{ in.} - (SID \ X \ \sin(15)) = (41 - 18.63) \text{in.} = 22.36 \text{ in.}$$

It should be noted that adjustments to default height values can be made according to region, such as by adjusting values proportionately according to height values that are characteristic of a particular patient population. Table 3 values, for example, would be applicable to a region or other patient population.

For auto-positioning calculation, the Table 2 values, suitable where patient height is 70 inches, can be scaled proportionately when patient height is less than or greater than 70 inches. Thus, for a patient of 62 inch height, a scaling factor of (62/70) can be applied to positioning calculations. The scaling factor(s) can also vary depending on patient age, sex, weight, and other factors, such as arthritis or other condition. Data that is indicative of patient height and can be acquired by processor 12 thus includes any of the actual measured or stored patient height value, a statistical height value from a regional or global patient population, and patient age, sex, weight, and other factors, such as arthritis or other condition. Default values can be used when the patient data that is indicative of patient height is unavailable or scanty. For example, if the only patient data that can be acquired indicates the sex of the patient, then the height used from stored statistical data for an adult of that sex would be used.

Referring back to FIG. 5, command signals transmitted to actuators 26 and 46 in a positioning step S830 of the FIG. 8 sequence effect the needed movement, to calculated positions, of transport mechanisms for x-ray source 20 and detector 16 and their supporting components corresponding to the identified exam type and patient. For generating the positioning signal(s) that actuate auto-positioning movement, processor 12 can use two positional data points: (i) the current position, or alternately the home position, and (ii) the position for imaging, and then determine a movement or repositioning of the x-ray source and detector from the current position to the imaging position. Step S830 can include a homing step in which transport apparatus for one or both the source and detector initially return to a default "home" position before repositioning to the calculated imaging position coordinates. Operator fine-tuning of the imaging position is then provided in a final adjustment step S840, and the actual final coordinates for imaging components are recorded. The final coordinates can then be stored in association with or referenced to patient identification data or patient image data and used to position the radiographic equipment for repeating the same type of exam for the patient at a future time. According to an alternate embodiment of the present disclosure, the actual stored coordinates can be used to train the positioning software, such as to adjust default values in order to better accommodate the local patient population, for example.

An image acquisition step S850 completes the imaging sequence in FIG. 8. The radiography apparatus 10 is then in position to acquire and display radiographic images of the patient, as well as to store acquired images in database 40.

Although embodiments of the present disclosure show an exemplary system that employs a ceiling mount, other equipment mounting arrangements are possible and are compatible with an embodiment of the present disclosure. Thus, for example, the x-ray source can be mounted to a pivoting arm or other device, rotatable about a number of axes. The x-ray source can be mounted as part of a floor-mounted system or a C-arm or U-arm system, for example. For a C-arm or U-arm system, the x-ray source and detector can maintain fixed positions relative to each other and only a single transport apparatus is needed.

Particular auto-position movements can be constrained following initial auto-positioning, depending on whether or not re-positioning established that different values should be applied. For example, if operator adjustment moves the x-ray source to a lower height, indicating a shorter patient, subsequent moves may be prevented or may use the lower height value. An error message can be displayed to the operator to indicate that an override of the calculated spatial position will be applied for subsequent images. Other rules restricting component movement may be applied, such as where the patient is in position and movement might be obstructed by or might inconvenience or endanger the patient.

For example, considering the C-Spine - LAO exposure example previously described, auto-positioning initially centered the detector at about 22.36 inch height. But if the 12 year-old patient is taller than average, subsequent manual adjustment is needed; the technician moves the x-ray source to a higher position, such as 25 inches for obtaining the C-spine LAO exposure. If the next image required in the exam for this patient is a C-Spine - RAO image, processor 12 (FIG. 5) adjusts according to the manually adjusted value, so that 25 inch height is used as the auto-position value for the particular patient unless otherwise manually adjusted. According to an embodiment of the present disclosure, subsequent images of other anatomy for this patient are similarly proportionately scaled according to manual adjustment. Continuing with the example of the same tall 12-year old, an adjusted auto-positioning value for skull imaging can be recomputed to be set 3 inches higher than the normally computed value, for example.

Technician adjustments can also be stored for table-top and other exposures that do not use a bucky. Adjustments following auto-positioning can be saved by the system for subsequent images for a particular patient setup.

The same type of adjustment described with relation to height is also applied for settings wherein the detector and x-ray source are angled. Considering the tall 12-year old in the above example, where the detector is to be tilted, an alternate value for the height of the detector would be computed before the tilt is applied.

Tabular statistical data is used to establish various starting parameters for imaging, such as initial positions for imaging an average adult, for example. This data is stored as factory default data and can be modified by personnel at the x-ray site, such as to adjust for different populations where values such as average height and, optionally, weight and age may vary from factory default values. Practices such as preferred angles for incident radiation may also be modified at the site, to account for local preferences. Default view types for different exams can be modified, as well as values for SID, and preferred bucky positions.

Embodiments of the present invention may include machine computations based on spatial coordinates in one, two, or three dimensions. Those skilled in the art will recognize that these calculations may be performed by data processing hardware that is provided with instructions for image data processing. Because such image manipulation systems are well known, the present description is directed more particularly to programs, algorithms and systems that execute the method of the present invention. Other aspects of such algorithms and systems, and data processing hardware and/or software for producing and otherwise processing the image signals may be selected from such systems, algorithms, components and elements known in the art. Given the description as set forth in the following specification, software implementation lies within the ordinary skill of those versed in the programming arts.

The stored instructions of such a software program may be stored in a computer readable storage medium, which may comprise, for example: magnetic storage media such as a magnetic disk or magnetic tape; optical storage media such as an optical disc, optical tape, or machine readable bar code; solid state electronic storage devices such as random access memory (RAM), or read only memory (ROM); or any other physical device or medium employed to store a computer program. Using such software, the present invention can be utilized on a data processing hardware apparatus, such as a computer system or personal computer, or on an embedded system that employs a dedicated data processing component, such as a digital signal processing chip.

Consistent with one embodiment, the present invention utilizes a computer program with stored instructions that execute on computer processor 12 that is configured to (i) accept an instruction or data that indicates an exam type for the patient; (ii) acquire patient data that is indicative of patient height; (iii) determine the present position of the first transport apparatus and the intended imaging position according to the accepted instruction and acquired patient data; and (iv) provide a first positioning signal to move the first transport apparatus from its present position to the intended imaging position. Computer processor 12 may also be configured to process the image data that is stored from detector 16 and accessed from an electronic memory. As can be appreciated by those skilled in the image processing arts, a computer program of an embodiment of the present invention can be utilized by a suitable, general-purpose computer system, such as a personal computer or workstation that acts as a control processor or other type of data processing hardware. However, many other types of computer systems can be used to execute the computer program of the present invention, including an arrangement of networked processors, for example. The computer program for performing the method of the present invention may be stored in a computer readable storage medium. This medium may comprise, for example; magnetic storage media such as a magnetic disk such as a hard drive or removable device or magnetic tape; optical storage media such as an optical disc, optical tape, or machine readable optical encoding; solid state electronic storage devices such as random access memory (RAM), or read only memory (ROM); or any other physical device or medium employed to store a computer program. The computer program for performing the method of the present invention may also be stored on computer readable storage medium that is connected to the processor by way of the internet or other network or communication medium. Those skilled in the art will further readily recognize that the equivalent of such a computer program product may also be constructed in hardware.

It is noted that the term "memory", equivalent to "processoraccessible memory" in the context of the present disclosure, can refer to any type of temporary or more enduring data storage workspace used for storing and operating upon stored data and accessible to a processor or computer system, including a database. The memory could be non-volatile, using, for example, a long-term storage medium such as electronic, magnetic, or optical storage. Alternately, the memory could be of a more volatile nature, using an electronic circuit, such as random-access memory (RAM) that is used as a temporary buffer or workspace by a microprocessor or other control logic processor device. Display data, for example, is typically stored in a temporary storage buffer that is directly associated with a display device and is periodically refreshed as needed in order to provide displayed data. This temporary storage buffer can also be considered to be a memory, as the term is used in the present disclosure. Memory is also used as the data workspace for executing and storing intermediate and final results of calculations and other processing. Computer-accessible memory can be volatile, non-volatile, or a hybrid combination of volatile and non-volatile types.

It is understood that the computer program product of the present invention may make use of various motion control and/or image manipulation algorithms and processes that are well known. It will be further understood that the computer program product embodiment of the present invention may embody algorithms and processes not specifically shown or described herein that are useful for implementation. Such algorithms and processes may include conventional utilities that are within the ordinary skill of the motion control and image acquisition and processing arts. Additional aspects of such algorithms and systems, and data processing hardware and/or software for producing and otherwise processing the images or co-operating with the computer program product of the present invention, are not specifically shown or described herein and may be selected from such algorithms, systems, hardware, components and elements known in the art.

The invention has been described in detail, and may have been described with particular reference to a suitable or presently preferred embodiment, but it will be understood that variations and modifications can be effected within the scope of the invention.

**Claims**

1. An auto-positioning apparatus for an x-ray imaging system, the auto-positioning apparatus comprising:

> a movable x-ray source (20);
> electronic storage containing a plurality of x-ray exam types, a plurality of patient heights and a plurality of x-ray source angles;
> a first transport apparatus (22) configured to automatically move and position the x-ray source (20) according to three spatial xyz coordinates relative to a detector (16) in response to receiving the three spatial xyz coordinates and an x-ray source angle, the first transport apparatus (22) comprising:
>
> > a ceiling mount (28) configured to move the x-ray source (20) along a plane substantially parallel with a ceiling along xz axes, and
> > a telescoping arm configured to raise or lower the x-ray source (20) along an y axis and to adjust an angle of the x-ray source (20); and
>
> a processor (12) communicatively coupled to the first transport apparatus (22), the processor (12) programmed to:
>
> > retrieve electronically stored data identifying one of the plurality of exam types with a corresponding x-ray source angle;
> > retrieve electronically stored patient data including data identifying a height of a patient to be imaged;
> > determine a current position of the x-ray source (20) along each of the three spatial xyz coordinates and determine a source imaging position along each of the three spatial xyz coordinates according to the retrieved data identifying the exam type with the corresponding x-ray source angle and the retrieved height of the patient; and
> > provide a first positioning signal identifying the three spatial xyz coordinates and the x-ray source angle to the first transport apparatus (22) to automatically move the x-ray source (20) from the determined current position to the determined source imaging position to start radiographically exposing the patient to be imaged.

2. The apparatus of claim 1, further comprising a second transport apparatus (32) configured to automatically move and position the detector (16) in response to a received second positioning signal, wherein the processor (12) is communicatively coupled to the second transport apparatus (32), and wherein the processor (12) is further programmed to:

> determine a current position of the detector (16) and determine a detector imaging position according to the retrieved data identifying the exam type and the patient data; and
> provide the second positioning signal to the second transport apparatus (32) to move the detector (16) from its determined current position to the detector imaging position.

3. The apparatus of claim 2, wherein the second transport apparatus (32) is further configured to position a bucky device.

4. The apparatus of claim 1, further comprising an operator interface configured to receive entry of the patient data.

5. The apparatus of claim 4, wherein the operator interface is further configured to receive entry of default height data representing a patient population to be imaged by the imaging system.

6. The apparatus of claim 1, wherein the first positioning signal additionally identifies an angle of the x-ray source.

7. The apparatus of claim 1, wherein the first transport apparatus (22) is further configured to automatically move and position the detector (16).

8. The apparatus of claim 1, wherein the processor (12) is further programmed to automatically electronically store data identifying a new position of the x-ray source (20) corresponding to one or more manual adjustments to the imaging position of the x-ray source (20) made by an operator of the apparatus.

9. A machine implemented method for acquiring an x-ray image of a patient, the method comprising:

retrieving electronic stored data that identifies an exam type, a patient type and an x-ray source angle and a patient height value associated with a patient, from a set of electronically stored exam types, values of patient height and x-ray source angles;
determining a source spatial location along each of the three xyz source spatial coordinates to position an x-ray source (20), the three spatial xyz coordinates determined according to the retrieved exam type, source angle and the retrieved patient height value;
providing a first signal to a ceiling mount (28) to automatically move and position the x-ray source (20) along xz axes parallel to a ceiling and to a telescoping arm to automatically raise or lower the x-ray source (20) along a vertical y axis and to adjust the position of the x-ray source (20) according to the determined source spatial location;
acquiring an image of the patient with the x-ray source (20) positioned at the determined source spatial location; and
displaying the x-ray image acquired with the x-ray source (20) positioned at the determined source spatial location along each of the three xyz source spatial coordinates.

10. The method of claim 9, further comprising:

determining detector spatial coordinates to position an x-ray detector (16) according to the identified exam type and the obtained height value; and
providing a second signal to an actuator (32) to automatically move and position the x-ray detector (16) according to the determined detector spatial coordinates.

11. The method of claim 9, wherein automatically positioning the x-ray source (20) comprises homing the x-ray source (20) at a reference home position.

12. The method of claim 9, further comprising recording an operator adjustment to the determined three xyz source spatial coordinates in an electronic memory.

13. The method of claim 9, further comprising recording spatial positions of one or more physical obstacles that may block movement of the x-ray source (20).

14. The method of claim 13, wherein determining spatial coordinates further comprises determining coordinates for moving the x-ray source (20) to avoid the one or more physical obstacles.

**Patentansprüche**

1. Eine Auto-Positionierungsvorrichtung für ein Röntgenbildgebungssystem, wobei die Auto-Positionierungsvorrichtung Folgendes aufweist:

eine bewegbare Röntgenquelle (20);
elektronischen Speicher, der eine Vielzahl von Röntgenuntersuchungstypen,
eine Vielzahl von Patientengrößen bzw. -höhen und eine Vielzahl von Röntgenquellenwinkeln enthält;
eine erste Transportvorrichtung (22), die eingerichtet ist, die Röntgenquelle (20) automatisch gemäß drei räumlichen xyz-Koordinaten relativ zu einem Detektor (16) zu bewegen und zu positionieren, und zwar als Reaktion auf ein Empfangen der drei räumlichen xyz-Koordinaten und eines Röntgenquellenwinkels, wobei die erste Transportvorrichtung (22) Folgendes aufweist:

eine Deckenhalterung (28), die eingerichtet ist, die Röntgenquelle (20) entlang einer Ebene, die im Wesentlichen parallel zu einer Decke ist,
entlang von xz-Achsen zu bewegen, und
einen Teleskoparm, der eingerichtet ist, die Röntgenquelle (20) entlang einer y-Achse anzuheben oder abzusenken und einen Winkel der Röntgenquelle (20) einzustellen; und
einen Prozessor (12), der kommunikativ mit der ersten Transportvorrichtung (22) gekoppelt ist, wobei der Prozessor (12) programmiert ist, zum:

Abrufen elektronisch gespeicherter Daten, die einen der Vielzahl von Untersuchungstypen mit einem entsprechenden Röntgenquellenwinkel identifizieren;
Abrufen elektronisch gespeicherter Patientendaten einschließlich Daten, die eine Höhe bzw. Größe eines abzubildenden Patienten identifizieren;
Bestimmen einer aktuellen Position der Röntgenquelle (20) entlang jeder der drei räumlichen xyz-Koordinaten und zum Bestimmen einer Quellenbildgebungsposition entlang jeder der drei räumlichen xyz-Koordinaten gemäß den abgerufenen Daten, die den Untersuchungstyp mit dem entsprechenden Röntgenquellenwinkel und der abgerufenen Höhe bzw. der Größe des Patienten identifizieren; und
Bereitstellen eines ersten Positionierungssignals, das die drei räumlichen xyz-Koordinaten und den Röntgenquellenwinkel identifiziert, und zwar an die erste Transportvorrichtung (22), um die Röntgenquelle (20) automatisch von der bestimmten aktuellen Position zu der bestimmten Quellenbildgebungsposition zu bewegen, um mit der Röntgenaufnahme bzw. der Röntgenbelichtung des abzubildenden Patienten zu beginnen.

2. Die Vorrichtung nach Anspruch 1, die ferner eine zweite Transportvorrichtung (32) aufweist, die eingerichtet ist, den Detektor (16) als Reaktion auf ein empfangenes zweites Positionierungssignal automatisch zu bewegen und positionieren,
wobei der Prozessor (12) kommunikativ mit der zweiten Transportvorrichtung (32) gekoppelt ist, und wobei der Prozessor (12) ferner programmiert ist zum:

Bestimmen einer aktuellen Position des Detektors (16) und zum Bestimmen einer Detektorbildgebungsposition gemäß den abgerufenen Daten, die den Untersuchungstyp und die Patientendaten identifizieren; und
Liefern des zweiten Positionierungssignals an die zweite Transportvorrichtung (32), um den Detektor (16) von seiner bestimmten aktuellen Position zur Detektorbildgebungsposition zu bewegen.

3. Die Vorrichtung nach Anspruch 2, wobei die zweite Transportvorrichtung (32) ferner eingerichtet ist, eine Buckybzw. Streustrahlen-Vorrichtung zu positionieren.

4. Die Vorrichtung nach Anspruch 1, die ferner eine Bedienerschnittstelle aufweist, die eingerichtet ist, eine Eingabe der Patientendaten zu empfangen.

5. Die Vorrichtung nach Anspruch 4, wobei die Bedienerschnittstelle ferner eingerichtet ist, eine Eingabe von Standard-Höhen- bzw. -größendaten zu empfangen, die eine von dem Bildgebungssystem abzubildende Patientenpopulation darstellen.

6. Die Vorrichtung nach Anspruch 1, wobei das erste Positionierungssignal zusätzlich einen Winkel der Röntgenquelle identifiziert.

7. Die Vorrichtung nach Anspruch 1, wobei die erste Transportvorrichtung (22) ferner eingerichtet ist, den Detektor (16) automatisch zu bewegen und zu positionieren.

8. Die Vorrichtung nach Anspruch 1, wobei der Prozessor (12) ferner programmiert ist, automatisch Daten elektronisch zu speichern, die eine neue Position der Röntgenquelle (20) identifizieren, die einer oder mehreren manuellen Anpassungen der Bildgebungsposition der Röntgenquelle (20) entsprechen, die von einem Bediener der Vorrichtung vorgenommen wurden.

9. Ein maschinenimplementiertes Verfahren zum Erfassen eines Röntgenbildes eines Patienten, wobei das Verfahren Folgendes aufweist:

Abrufen von elektronisch gespeicherten Daten, was einen Untersuchungstyp, einen Patiententyp und einen Röntgenquellenwinkel und

einen Wert für eine Patientenhöhe bzw. größe, die mit einem Patienten assoziiert ist, aus einem Satz von elektronisch gespeicherten Untersuchungstypen, Werten von Patientenhöhen bzw. -größen und Röntgenquellenwinkeln identifiziert;

Bestimmen eines räumlichen Quellenortes entlang jeder der drei räumlichen xyz-Quellen-Koordinaten, um eine Röntgenquelle (20) an dem bestimmten räumlichen Quellenort zu positionieren, wobei die drei räumlichen xyz-Koordinaten gemäß dem abgerufenen Untersuchungstyp und dem abgerufenen Höhen- bzw. Größenwert des Patienten bestimmt werden;

Bereitstellen eines ersten Signals an eine Deckenhalterung (28) zum automatischen Bewegen und Positionieren der Röntgenquelle (20) entlang von xz-Achsen parallel zu einer Decke und zu einem Teleskoparm, um die Röntgenquelle (20) entlang einer vertikalen y-Achse automatisch anzuheben oder abzusenken und um eine Position der Röntgenquelle (20) gemäß dem bestimmten räumlichen Quellenort einzustellen;

Erfassen eines Bildes des Patienten mit der Röntgenquelle (20), die am bestimmten räumlichen Quellenort positioniert ist; und

Anzeigen des Röntgenbildes, das mit der an dem bestimmten räumlichen Quellenort positionierten Röntgenquelle (20) aufgenommen wurde, und zwar entlang jeder der drei räumlichen xyz-Quellen-Koordinaten.

10. Das Verfahren nach Anspruch 9, das ferner Folgendes aufweist:

Bestimmen von räumlichen Detektor-Koordinaten zum Positionieren eines Röntgendetektors (16) gemäß dem identifizierten Untersuchungstyp und dem erhaltenen Höhen- bzw. Größenwert; und

Bereitstellen eines zweiten Signals an einen Aktor (32), um den Röntgendetektor (16) gemäß den bestimmten räumlichen Detektor-Koordinaten automatisch zu bewegen und zu positionieren.

11. Das Verfahren nach Anspruch 9, wobei automatisches Positionieren der Röntgenquelle (20) Homing bzw. Ansteuern der Röntgenquelle (20) in einer Referenz-Ansteuerungsposition aufweist.

12. Das Verfahren nach Anspruch 9, das ferner Aufzeichnen einer Bedieneranpassung zu den ermittelten drei räumlichen xyz-Quellen-Koordinaten in einem elektronischen Speicher aufweist.

13. Das Verfahren nach Anspruch 9, das ferner Aufzeichnen der räumlichen Positionen von einem oder mehreren physikalischen Hindernissen aufweist, die die Bewegung der Röntgenquelle (20) blockieren können.

14. Das Verfahren nach Anspruch 13, wobei Bestimmen räumlicher Koordinaten ferner Bestimmen von Koordinaten zum Bewegen der Röntgenquelle (20) aufweist, um das eine oder die mehreren physikalischen Hindernisse zu vermeiden.

**Revendications**

1. Appareil de positionnement automatique destiné à un système d'imagerie à rayons X, l'appareil de positionnement automatique comprenant :

une première source de rayons X mobile (20) ;

un stockage électronique contenant une pluralité de types d'examen aux rayons X, une pluralité de tailles de patient et une pluralité d'angles de la source de rayons X ;

un premier appareil de transport (22) configuré pour déplacer et positionner automatiquement la source de rayons X (20) en fonction de trois coordonnées spatiales xyz par rapport à un détecteur (16) en réponse à la réception des trois coordonnées spatiales xyz et d'un angle de la source de rayons X, le premier appareil de transport (22) comprenant :

un montage au plafond (28) configuré pour déplacer la source de rayons X (20) le long d'un plan sensiblement parallèle à un plafond le long d'axes xz, et

un bras télescopique configuré pour lever ou abaisser la source de rayons X (20) le long d'un axe y et pour régler un angle de la source de rayons X (20) ; et

un processeur (12) couplé en communication au premier appareil de transport (22), le processeur (12) étant programmé pour :

récupérer des données stockées électroniquement identifiant un type parmi la pluralité de types d'examen avec un angle correspondant de la source de rayons X ;

récupérer des données de patient stockées électroniquement comportant des données identifiant une taille d'un patient à imager ;

déterminer une position courante de la source de rayons X (20) le long de chacune des trois coordonnées spatiales xyz et déterminer une position d'imagerie de la source le long de chacune des trois coordonnées spatiales xyz en fonction des données récupérées identifiant le type d'examen avec l'angle correspondant de la source de rayons X et la taille récupérée du patient ; et

fournir un premier signal de positionnement identifiant les trois coordonnées spatiales xyz et l'angle de la source de rayons X au premier appareil de transport (22) pour déplacer automatiquement la source de rayons X (20) depuis la position courante déterminée à la position d'imagerie source déterminée pour déclencher l'exposition radiographique du patient à imager.

2. Appareil selon la revendication 1, comprenant en outre un deuxième appareil de transport (32) configuré pour déplacer et positionner automatiquement le détecteur (16) en réponse à un deuxième signal de positionnement reçu, dans lequel le processeur (12) est couplé en communication au deuxième appareil de transport (32), et dans lequel le processeur (12) est en outre programmé pour :

déterminer une position courante du détecteur (16) et déterminer une position d'imagerie du détecteur en fonction des données récupérées identifiant le type d'examen et les données du patient ; et

fournir le deuxième signal de positionnement au deuxième appareil de transport (32) pour déplacer le détecteur (16) depuis sa position courante déterminée à la position d'imagerie du détecteur.

3. Appareil selon la revendication 2, dans lequel le deuxième appareil de transport (32) est en outre configuré pour positionner un dispositif de Bucky.

4. Appareil selon la revendication 1, comprenant en outre une interface opérateur configurée pour recevoir une entrée des données du patient.

5. Appareil selon la revendication 4, dans lequel l'interface opérateur est en outre configurée pour recevoir une entrée de données de taille par défaut représentant une population de patients à imager par le système d'imagerie.

6. Appareil selon la revendication 1, dans lequel le premier signal de positionnement identifie en outre un angle de la source de rayons X.

7. Appareil selon la revendication 1, dans lequel le premier appareil de transport (22) est en outre configuré pour déplacer et positionner automatiquement le détecteur (16).

8. Appareil selon la revendication 1, dans lequel le processeur (12) est en outre programmé pour stocker automatiquement électroniquement des données identifiant une nouvelle position de la source de rayons X (20) correspondant à un ou plusieurs réglages manuels de la position d'imagerie de la source de rayons X (20) effectués par un opérateur de l'appareil.

9. Procédé mis en œuvre par machine destiné à acquérir une image de rayons X d'un patient, le procédé comprenant :

la récupération de données stockées électroniquement qui identifient un type d'examen, un type de patient et un angle de la source de rayons X et une valeur de taille de patient associée à un patient, à partir d'un ensemble de types d'examen stockés électroniquement, de valeurs de taille de patient et d'angle de la source de rayons X ;

la détermination d'un emplacement spatial de la source le long de chacune des trois coordonnées spatiales xyz pour positionner une source de rayons X (20), les trois coordonnées spatiales xyz étant déterminées en fonction du type d'examen récupéré et de la valeur de taille de patient récupérée ;

la fourniture d'un premier signal à un montage au plafond (28) pour déplacer et positionner automatiquement la source de rayons X (20) le long des axes xz parallèles à un plafond et à un bras télescopique pour lever ou abaisser automatiquement la source de rayons X (20) le long d'un axe vertical y et pour régler la position de la source de rayons X (20) en fonction de la position spatiale déterminée de la source ;

l'acquisition d'une image du patient avec la source de rayons X (20) positionnée au niveau de la position spatiale déterminée de la source ; et

l'affichage de l'image à rayons X acquise avec la source de rayons X (20) positionnée au niveau de la position spatiale déterminée de la source le long de chacune des trois coordonnées spatiales xyz.

10. Procédé selon la revendication 9, comprenant en outre :

la détermination de coordonnées spatiales de détecteur pour positionner un détecteur de rayons X (16) en fonction du type d'examen identifié et de la valeur de taille obtenue ; et

la fourniture d'un deuxième signal à un actionneur (32) pour déplacer et positionner automatiquement le détecteur de rayons X (16) en fonction des coordonnées spatiales déterminées du détecteur.

11. Procédé selon la revendication 9, dans lequel le positionnement automatique de la source de rayons X (20) comprend la mise au repos de la source de rayons X (20) à une position de repos de référence.

12. Procédé selon la revendication 9, comprenant en outre l'enregistrement d'un réglage de l'opérateur aux trois coordonnées spatiales déterminées xyz dans une mémoire électronique.

13. Procédé selon la revendication 9, comprenant en outre l'enregistrement de positions spatiales d'un ou de plusieurs obstacles physiques qui peuvent bloquer le mouvement de la source de rayons X (20).

14. Procédé selon la réclamation 13, dans lequel la détermination des coordonnées spatiales comprend en outre la détermination de coordonnées pour déplacer la source de rayons X (20) pour éviter lesdits un ou plusieurs obstacles physiques.

FIG. 1

*FIG. 2*

*FIG. 3*

EP 3 253 291 B1

20

16

10

30

14

**FIG. 4**

**FIG. 5**

Wall Stand

Bucky 14

Detector 16

z

15°

72" SID

Detector Center 41" from Floor

Focal Spot

Tube Head

20

x

y

*FIG. 6*

EP 3 253 291 B1

**FIG. 7**

**FIG. 8**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2011122995 A1
- JP 2013154146 A

- WO 2014033614 A1